# EUROPEAN PATENT APPLICATION

(11) **EP 1 655 025 A1**
(43) Date of publication of application: **10.05.2006**
(21) Application number: 05109879.6
(22) Date of filing: 24.10.2005
(51) Int. Cl.: A61K 9/70, A61K 35/02

(54) **Patch containing a substance selected from tars having reducing characteristics in dermatology**

(30) Priority: 04.11.2004 IT MI20042111
(71) Applicant: BIOFARMITALIA S.p.A., 20144 Milano (IT)
(72) Inventor: Pinna, Marco, 21056, INDUNO OLONA (VA) (IT); Pinna, Fausto, 20050, LESMO (MI) (IT)
(74) Representative: Frignoli, Luigi

(57) **Abstract**

A patch consisting of a porous or occlusive woven, non-woven or plastic support, to one face of which is applied a skin adhesive polymer layer containing tars used as reducers or resolvents in dermatology, in particular ichthyol, able to gradually release the active substances in order to maximize their therapeutic properties.

## Description

The present invention provides a transpirable or non-transpirable patch or the like, one surface of which is at least partially covered by a skin-adhesive polymer layer able to retain sufficient doses of tars used as reducers or resolvents in dermatology, such as ichthyol or sulfonated ichthyol, and to suitably release their therapeutically active substances.

Tars used as reducers in dermatology are classically divided into: vegetable tars, bituminous tars such as ichthyol and ichthammol, and tars from fossil coal (coal tar). Currently, vegetable tars are little prescribed because application methods used up to now can give rise to irritation, and are hence applied only to poorly sensitive skin for just a few days, so avoiding treatment of irritable areas. Ichthyol and ichthammol have been progressively abandoned as they are often sensitizing and difficult to use. Ichthyol is a product derived from the dry distillation of an oil shale and has similar characteristics to coal tar, it containing 10% of sulphur.

It has long been used for its anti-inflammatory, antiseptic, vasoconstricting and anti-pruritic action. It is soluble in water and is therefore often formulated as aqueous preparations for psoriasis of the face, for atopic dermatitis and acne. The odour of sulphur-containing preparations is very strong and unpleasant. Applying the ointment is difficult and uncomfortable as it is stringy. After use the area has to be washed to remove residues and any stains left on the skin following treatment. The posology required for these cutaneous manifestations is several applications during the day. In frequent cases ichthyol is used to clear up anal fistulas by applications of ointments, creams or gauzes impregnated with ichthyol based solutions which stain, dirty and irritate all the surrounding area and the underwear, to the extent of seeping onto the outer clothing with its evident complications. Moreover once the abscess has matured, the discharge of mucopurulent material, because it is not retained, spreads out and compromises the entire area.

Ichthyol can give rise to irritation and sensitivity if applied to particularly delicate areas, hence it is important to have a pharmaceutical form, such as a patch, which can ensure a constant release of the active substance, but without ever reaching a concentration such as to be poorly tolerated by the skin.

The main object of the present invention is to provide a patch, one surface of which carries a polymer layer applicable to human skin by virtue of its adhesivenes containing sufficient doses of ichthyol or ichthyol sulfonate or tumenol, coal tar or another tar used as a reducer in dermatology, which is able to treat the specific afflicted area with a limited quantity of substance, without invading the surrounding areas so as to avoid intolerances and sensitizations, is able to release the substance in a gradual and constant manner with time, and is able to retain and absorb the mucopurulent material which escapes after the pimple, fistula or abscess has cleared up.

These objects are achieved by a patch characterized by a layer of skin-adhesive polymer applied to one of its faces, which contains at least one substance chosen from tars used as reducers in dermatology with cosmetic or therapeutic activity, and consisting more specifically of a flexible support of non-woven fabric, cotton or another fabric or plastic material, to at least one surface of which a layer of adhesive polymer is applied, chosen from synthetic groups, for example acrylic in a water or solvent base, vinyl in a water or solvent base, polyurethane, resins of natural or synthetic origin, polyacrylates, or natural polymers such as gums, polyvinyl alcohols, cellulose, carrageenan, alginates, etc. within which polymer a substance is dispersed chosen from tars used as reducers or resolvers in dermatology in a percentage variable between 0.1 and 60% of its final weight on the patch, with the possible addition of between 0% and 10% of a substance of vegetable origin comprising essential oils and aromatic extracts; and between 0% and 10% of a skin-compatible component chosen from the group comprising soothing substances, skin repairers, cicatrizants, anti-inflammatories, antiseptics and bactericides, the percentages being by weight.

The polymer can also contain between 0.0% and 5% by weight of a useful but non-essential compound chosen from the group formed from wetting solvents, preservatives, emulsifiers, stabilizers, solubilizers, surfactants and colorants.

Within the patch the polymer is preferably present in a quantity from 70% to 97% by weight, the substance selected from tars used as reducers, preferably pure or sulfonated ichthyol or tumenol being between 1% and 30% by weight, soothing substances, skin repairers, cicatrizants, anti-inflammatory, antiseptic or bactericide being between 0% and 5%, and between 0.0% and 2% by weight of a component chosen from the group formed from wetting solvents, preservatives, emulsifiers, stabilizers, solubilizers, surfactants and colorants, the percentages being by weight.

The preferred polymers are chosen from the group comprising acrylic, polyacrylic, polyurethanic polymers, synthetic or natural resins, polyvinyl alcohol, sodium alginate, calcium alginate, carboxymethylcellulose, sodium polyacrylate and polyvinylpyrrolidone.

Some non-limiting embodiments are described hereinafter to further clarify the understanding of the nature, shape and structure and of the method of obtaining the patch.

### EXAMPLE 1

### Preparation of an ichthyol based patch for treating anal fistulas

8.7 kg of a non-crosslinked solvent acrylic adhesive (for example Duro-tak 387-2353 adhesive by National Starch & Chemical) and 323 grams of pure ichthyol are introduced into a container under cold conditions and mixed with a propeller stirrer until a uniform mass is obtained. The mass is left to stand to enable any air bubbles to escape. Using a patch spreading machine and with the aid of a compressed air pump, the mixture is fed into the rotating roller doctor blade with the doctor blade thickness adjusted to about 300 micron. The mixture is filmed onto a siliconized polyester which passes through 4 oven stations, the first set at 40°C, the second at 50°C, the third at 70°C and the fourth at 80°C, at a rate of 8 metres per minute. At the oven exit the film is completely free of solvents, which have evaporated within the oven stations, the body of the adhesive mass is blackish in colour showing the presence of 10% pure ichthyol, the weight of the adhesive mass being about 90 grams per square metre. The polyester on which the adhesive film was spread and dried is bonded to a polyester and viscose non-woven fabric of 100 grams per square metre and rewound onto a bobbin. The result is that the adhesive film grips the non-woven fabric to form an adhesive fabric containing 10% ichthyol protected by the siliconized polyester. Round patches of 4 centimetres diameter are then punched from the obtained bobbin and packaged. These patches were tested by HPLC to verify the quantity of pure ichthyol present, in each patch a quantity of 11.3 mg of ichthyol being found, equal to 10% of the total adhesive mass.

Tests subsequently undertaken by organized clinical structures on persons with anorectal fistulas or abscesses demonstrated the absolute effectiveness of the product. The abscess was found to re-absorb with complete resolution of pain and tissue symptomology. In the space of 8-10 hours there is a progressive recession of characteristic symptoms: anoperineal pain and swelling, irritation and reddening of the perineal skin (itching, burning, and pus).

### EXAMPLE 2

### Preparation of an ichthyol based patch for treating anal fistulas.

20 kg of a crosslinked solvent acrylic adhesive with average viscosity 2500/3000 mPa.s (for example Duro-tak 387-2054 adhesive by National Starch and Chemical) and 5 kg of solvent acrylic adhesive with 700-800 mPa.s low viscosity were introduced under cold conditions into a container then mixed slowly for 20 minutes with a vertical agitator. While slowly agitating, 2100 grams of pure ichthyol (ammonium sulfoichthyolate), 247 g of tumenol and 123 grams of fluid Aloe Vera extract are introduced as a thin stream. Mixing continues slowly until a uniform mass is obtained then left to stand to release any air bubbles. Using a patch spreading machine and with the aid of a compressed air pump, the mixture is fed into the rotating roller doctor blade with the doctor blade thickness adjusted to about 320 micron. The mixture is filmed onto a siliconized polyester which passes through 4 oven stations, the first set at 40°C, the second at 50°C, the third at 80°C and the fourth at 90°C, at a rate of 8 metres per minute. At the oven exit the film is completely free of solvents, which have evaporated within the oven stations, the body of the adhesive mass is blackish in colour showing the presence of 8% pure ichthyol, 1% tumenol, 0.5% Aloe Vera, the weight of the adhesive mass being about 100 grams per square metre. The polyester on which the adhesive film has been spread and dried is bonded to a micro-perforated corona treated polythene film of 90 g/m² and rewound on a bobbin. The result is that the adhesive film grips the micro-perforated polythene to form an adhesive fabric containing 8% ichthyol, 1% tumenol and 0.5% Aloe Vera protected by the siliconized polyester. Round patches of 4 centimetres diameter are then punched from the obtained bobbin and packaged.

These patches were tested by HPLC to verify the quantity of pure ichthyol present, in each patch a quantity of 8.9 mg of ichthyol being found, equal to 8% of the total adhesive mass.

### EXAMPLE 3

### Preparation of an ichthyol based patch for treating inflammatory changes of the skin.

28 kg of demineralised water, 0.120 kg of parabens (preservatives), 4.4 kg of polyvinyl alcohol and 0.5 kg of carboxymethyl cellulose are introduced into a mixer heated to 70°C to obtain a uniform mass (Phase A) which is cooled to ambient temperature and then poured into a kneader into which Phase C, formed of 3 kg of ichthyol (ammonium sulfoichthyol), 2.0 kg of carboxymethyl beta-glucan (having cicatrising action), and 18 kg of sodium alginate are added under cold conditions (over a period of about 15-20 minutes). The mixture is agitated in both directions for 15-20 minutes, after which a Phase B, prepared separately by mixing 1.8 kg of demineralised water and 0.5 kg of sodium tetraborate in a steel container at a temperature of 20-30°C until completely dissolved, is slowly added in a thin stream to give a gel whose viscosity can be increased by increasing the quantity of Phase B or decreased by increasing the quantity of Phase A.

The gel is spread by means of a rotating two roller doctor blade onto a web of non-woven fabric formed from viscose fibres (50%) and polypropylene fibres (50%), with a weight of 150 g/m², a thickness of 200 micron and a density of 87 g/dm³.

The protection sheet is a 75 g/m² siliconized polyester sheet. The composite web which leaves the rollers is transported to a machine where it is cut and punched, for example into the form of a 10 x 15 cm rectangular patch saturated throughout.

The patch thus obtained is found to be particularly effective for applications in very irritated areas, in that the refreshing action of the gel offers an immediate soothing and calming effect, the therapeutic action being attributable to the ichthyol and to the skin repairing activity (mainly due to the carboxymethyl beta-glucan).

### EXAMPLE 4

### Preparation of an ichthyol based patch for treating anal fistulas.

6 kg of a styrene and isopropene based copolymer (for example KRATON D-1 161 N by Shell Chemicals), 2 kg of an alpha pinene terpene resin (for example DERCOLYTE A 115 by DRT), 10 kg of toluene and 4 kg of MEK are introduced into a container and mixed slowly for 20 minutes with a vertical agitator. 500 grams of pure ichthyol (ammonium sulfoichthyol) are added as a thin stream under slow agitation. Mixing is continued slowly until a uniform mass is obtained. The mass is left to stand to release any air bubbles. Using a patch spreading machine and with the aid of a compressed air pump, the mixture is fed into the rotating roller doctor blade with the doctor blade thickness adjusted to about 320 micron. The mixture is filmed onto a siliconized polyester which passes through 4 oven stations, the first set at 40°C, the second at 50°C, the third at 80°C and the fourth at 90°C, at a rate of 8 metres per minute. At the oven exit the film is completely free of solvents, which have evaporated within the oven stations, the body of the adhesive mass is blackish in colour showing the presence of 10% pure ichthyol, the weight of the adhesive mass being about 100 grams per square metre. The polyester on which the adhesive film was spread and dried is bonded to pure viscose non-woven fabric of 100 g/m² and rewound on a bobbin. The result is that the adhesive film grips the unwoven fabric to form an adhesive fabric containing 10% ichthyol protected by the siliconized polyester. Round patches of 4 centimetres diameter are punched from the bobbin obtained and packaged

These patches were tested by HPLC to verify the quantity of pure ichthyol present, in each patch a quantity of 7 mg of ichthyol being found, equal to 10% of the total adhesive mass.

## Claims

1. A patch applicable to the skin, **characterized in that** a layer of skin-adhesive polymer is applied to one of its faces, said layer containing at least one substance chosen from tars used as reducers in dermatology with cosmetic or therapeutic activity.

2. A patch as claimed in claim 1 **characterized in that** the substance chosen from coal tars is ichthyol.

3. A patch as claimed in claim 1, **characterized in that** the substance chosen from coal tars is tumenol.

4. A patch as claimed in claim 1, **characterized in that** the substance chosen from coal tars is coal tar from fossil coal.
